# EUROPEAN PATENT APPLICATION

(11) **EP 4 696 672 A1**
(43) Date of publication of application: **18.02.2026**
(21) Application number: 24194939.5
(22) Date of filing: 16.08.2024
(51) Int. Cl.: C07C 17/25, C07C 21/21, C07C 21/20

(54) **PROCESS FOR THE SYNTHESIS OF 2-CHLORO-1,3-BUTADIENE AND 2,3-DICHLORO-1,3-BUTADIENE**

(71) Applicant: ARLANXEO Deutschland GmbH, 41540 Dormagen (DE); Friedrich-Alexander-Universität Erlangen-Nürnberg, 91054 Erlangen (DE)
(72) Inventor: Schmidt, Roland, 41540 Dormagen (DE); Michels, Kevin, 41540 Dormagen (DE); Kirchhoff, Jörg, 41540 Dormagen (DE); Wasserscheid, Peter, 91058 Erlangen (DE); Schulz, Peter, 91058 Erlangen (DE)

(57) **Abstract**

The invention relates to a process for the preparation of a halogenated 1,3-butadiene by dehydrohalogenation of a halogenated 1-butene. Preferably, the process is for the preparation of 2-chloro-1,3-butadiene (CP) from 3,4-dichloro-1-butene (3,4-DBN), or for the preparation of 2,3-dichloro-1,3-butadiene (DCB) from 2,3,4-trichloro-1-butene (TCB). The dehydrohalogenation is performed in the presence of an organic base that forms a salt with eliminated hydrohalogenic acid and provides a low melting halide salt, preferably an organic salt melting below 100 °C, that is an ionic liquid. Preferably, halogenated 1,3-butadiene is separated from the reaction mixture by in-situ distillation. Organic base is recovered from the low melting organic salt by e.g. heating and release of hydrohalogenic acid. After purification, recovered organic base is recycled into the process.

## Description

The invention relates to a process for the preparation of a halogenated 1,3-butadiene by dehydrohalogenation of a halogenated 1-butene. Preferably, the process is for the preparation of 2-chloro-1,3-butadiene (CP) from 3,4-dichloro-1-butene (3,4-DBN), or for the preparation of 2,3-dichloro-1,3-butadiene (DCB) from 2,3,4-trichloro-1-butene (TCB). The dehydrohalogenation is performed in the presence of an organic base that forms a salt with eliminated hydrohalogenic acid and provides a low melting halide salt, preferably an organic salt melting below 100 °C, that is an ionic liquid. Preferably, halogenated 1,3-butadiene is separated from the reaction mixture by in-situ distillation. Organic base is recovered from the low melting organic salt by e.g. heating and release of hydrohalogenic acid. After purification, recovered organic base is recycled into the process.

2-Chloro-1,3-butadiene (CP), 2,3-dichloro-1,3-butadiene (DCB) and related monomers are used for manufacturing chloroprene polymer (CR). 2-Chloro-1,3-butadiene (CP) and 2,3-dichloro-1,3-butadiene (DCB) can be prepared by dehydrochlorination of 3,4-dichloro-1-butene (3,4-DBN) and 2,3,4-trichloro-1-butene (TCB), respectively:

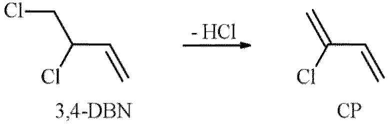

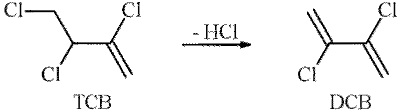

It is known to prepare these monomers in a biphasic process (organic/caustic) by phase transfer catalysis using an organic ammonium chloride NR₄⁺Cl⁻ as catalyst. The catalyst is subsequently decomposed in the aqueous caustic phase to form an alkali salt solution from which chlorine is recuperated.

For example, US 4,418,232 relates to a process for dehydrohalogenating a halogenated hydrocarbon to an ethylenically unsaturated product in the presence of a phase-transfer catalyst. US 4,629,816 discloses a process wherein 2,3-Dichlorobutadiene-(1,3) is obtained from 2,3,4-trichlorobutene-1 by dehydrohalogenation in the presence of a phase transfer catalyst, an inhibitor and oxygen.

It is also known to use ionic liquids in a wide variety of different industrial processes. For details, reference is made to N.V. Plechkova et al., Chem. Soc. Rev., 2008, 37, 123-150; and A.J. Greer et al., Molecules 2020, 25, 5207, 1-31.

WO 2002/000639 relates to a method for separating acids from chemical reaction mixtures by means of an auxiliary base. Said auxiliary base b) forms a salt with the acid, which is liquid at temperatures at which the valuable product is not significantly decomposed during separation, and c) the salt of the auxiliary base and the valuable product or the solution of the valuable product form two immiscible fluid phases in a suitable solvent.

WO 2002/094740 relates to heterogeneous processes for the hydro-dehalogenation of a compound containing at least one C-Cl, C-Br or C-1 bond. The processes comprise reacting said compound with a hydrogenating agent and a heterogeneous hydrogenation catalyst in the presence of an ionic liquid.

WO 2003/062251 relates to a method for producing amino dihalophosphines, diamino halo-phosphines, triamino phosphines, phosphite diamides, amino phosphines, diamino phosphines, phosphite amide halogenides, and amino phosphine halogenides by separating an acid in the presence of an auxiliary base. Said auxiliary base b) forms a salt with an acid, which is liquid at temperatures at which the valuable product is not significantly decomposed during separation of the liquid salt, and c) the salt of the auxiliary base and the valuable product or the solution of the valuable product form two immiscible phases in a suitable solvent.

WO 2005/061416 relates to a method for isolating acids from chemical reaction mixtures by means of an auxiliary base, whereby this auxiliary base: b) forms a salt with the acid, which is liquid at temperatures at which the valuable product is not significantly decomposed during the isolation of the liquid salt, and; c) the salt of the auxiliary base, together with the valuable product or with the solution of the valuable product, forms, in a suitable solvent, two non-mixable liquid phases.

BASF's patented BASIL process (Biphasic Acid-Scavenging utilizing Ionic Liquids) is probably the most widely known and recognized example of industrial implementation of an ionic liquid-based process. The BASIL process is commercially important because of the significant process improvements realized versus the prior process. In the latter, acid scavenging in the reaction was achieved using alkylamines, like triethylamine, forming the protonated trialkyl ammonium halide salt. This product is a thick, dense, insoluble slurry that is difficult to handle and remove from the reactor, resulting in inefficiencies and the need for a processing solvent. By utilizing the ionic liquid precursor, 1-methylimidazole, as an alternative to triethylamine, the acid reaction forms 1-methylimidazolium chloride, a protic ionic liquid with a low melting point (75 °C) which separates and forms a biphasic system in the reaction vessel. The lower phase being comprised of pure ionic liquid can readily be removed, resulting in easy isolation of the upper phase product. Furthermore, the ionic liquid can be recycled by deprotonation, regenerating the 1-methylimidazole reactant (M.A. Benvenuto et al., Green Chemistry in Industry, DeCrruyter, 2018, pages 44-45).

The processes of the prior art for producing halogenated 1,3-butadienes are not satisfactory in every respect because they have high complexity and high energy demand. The processes suffer from low reactivity (e.g., space-time-yield: 2-chloro-1,3-butadiene (CP): 0.36 t/h·m³; 2,3-dichloro-1,3-butadiene (DCB): 0.04 t/h·m³). Further, in the course of the preparation due to the employed phase transfer catalyst, a suspension and a sludge are formed that are difficult to separate. Still further, regeneration of the caustic solution is costly.

The processes of the prior art rely upon complex chemistry for dehydrohalogenation based on two phases with the need of a phase transfer agent (without recycling the catalyst). The resulting aqueous phase needs to be treated internally by complex equipment and high operational costs. The processes are moreover very sensitive to changing conditions and frequently lead to process issues. The wastewater carries an increased salt freight making wastewater treatment difficult and laborious.

There is a demand for processes for the preparation of halogenated 1,3-butadienes from halogenated 1-butenes that overcome at least some of the drawbacks of the prior art.

It is an object of the invention to provide processes for the preparation of halogenated 1,3-butadienes from halogenated 1-butenes that have advantages compared to the processes of the prior art. The processes should preferably be simplified, more efficient and safer.

This object has been achieved by the subject-matter of the patent claims.

It has been surprisingly found that the above advantages can be achieved by a different reaction scheme using an organic base that forms low melting organic halide salts, preferably salts melting below 100 °C, that is an ionic liquid. The suspension forming phase transfer catalyst of the prior art is avoided while simultaneously providing a process that is more efficient due to the reduction of in-between steps.

Further, it has been surprisingly found that production for both 2-chloro-1,3-butadiene (CP) and 2,3-dichloro-1,3-butadiene (DCB) can be streamlined with a simplified chemistry at lower equipment and energy costs. This is basically related to wastewater or brine generation and handling. The consumption of fresh water is avoided.

Compared to the processes of the prior art, the process according to the invention has at least some, preferably all of the following advantages:
(i) recycle of auxiliary raw materials, e.g., organic base and halide or pseudo-halide salt or ionic liquid can be recycled; cleaved hydrohalogenic acid can be recovered and used e.g. in other process steps like coagulation, for recovering the halogen in a subsequent step or as a value stream that it sold externally;
(ii) lower volume of raw material supply and reduced logistic complexity, e.g., with respect to a phase transfer agent, sodium hydroxide, and hydrochloric acid;
(iii) energy savings e.g. with respect to steam for saline and steam for wastewater stripping;
(iv) environmental and occupational benefits, e.g., with respect to improved safety due to less holdup within reactors; less fresh water consumption for reaction; elimination of wastewater production and salt freight; no issue regarding total organic carbon (TOC) in wastewater; reduction of waste e.g. phase transfer agent sludge; replacement of hazardous materials; and less manual work for filling of chemicals and process aids (e.g. phase transfer catalyst);
(v) less operational complexity, e.g., no caustic wastewater treatment; difficult and complex to operate, many issues with sludge and sludge treatment; smaller Dehydrohalogenation equipment, less maintenance efforts due to clean process;
(vi) increased space-time-yield;
(vii) optimization of synthesis.

A first aspect of the invention relates to a process for the preparation of a halogenated 1,3-butadiene of general formula (I) 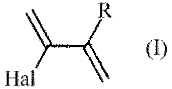 wherein
R means -H or Hal; and
Hal independently means -Cl, -Br or -I; preferably -Cl;
said process comprising the steps of:
(a) providing a reaction mixture containing or essentially consisting of
   - a halogenated 1-butene of general formula (II) 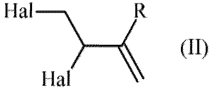 wherein
      R means -H or Hal; and
      Hal independently means -Cl, -Br or -I; preferably -Cl;
   - an organic base; and
   - optionally a catalyst;
(b) eliminating hydrohalogenic acid from the halogenated 1-butene to produce the halogenated 1,3-butadiene; and forming a salt of the eliminated hydrohalogenic acid with the organic base thereby obtaining a low melting halide salt, preferably an ionic liquid, in the reaction mixture;
(c) optionally, separating at least a portion of the halogenated 1,3-butadiene from the reaction mixture by in-situ distillation;
(d) separating the reaction mixture into
   - a product composition containing halogenated 1,3-butadiene and optionally residual halogenated 1-butene; and
   - a salt composition containing salt of the eliminated hydrohalogenic acid with organic base and optionally residual organic base;
(e) optionally, purifying the halogenated 1,3-butadiene contained in the product composition; optionally together with the portion of the halogenated 1,3-butadiene optionally separated in step (c);
(f) cleaving the salt to release hydrohalogenic acid from the salt and separating the salt composition into
   - a hydrohalogenic acid composition containing released hydrohalogenic acid; and
   - an organic base composition containing organic base;
(g) optionally, purifying the organic base contained in the organic base composition thereby obtaining purified organic base; and
(h) recycling the organic base composition or the purified organic base to the reaction mixture.

Preferably, the halogenated 1-buten is 3,4-dichloro-1-butene (3,4-DBN) and the halogenated 1,3-butadiene is 2-chloro-1,3-butadiene (CP):

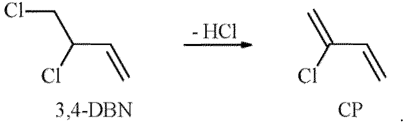

Preferably, the halogenated 1-buten is 2,3,4-trichloro-1-butene (TCB) and the halogenated 1,3-butadiene is 2,3-dichloro-1,3-butadiene (DCB):

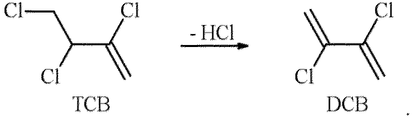

Preferably, the reaction mixture has a water content of not more than 8 wt.-%, preferably not more than 7 wt.-%, more preferably not more than 6 wt.-%, still more preferably not more than 5 wt.-%, yet more preferably not more than 4 wt.-%, even more preferably not more than 3 wt.-%, most preferably not more than 2 wt.-%, and in particular not more than 1 wt.-%, in each case relative to the total weight of the reaction mixture, or is essentially free of water; preferably the total process is performed essentially in the absence of water.

Preferably, the reaction mixture has a solvent content of not more than 8 wt.-%, preferably not more than 7 wt.-%, more preferably not more than 6 wt.-%, still more preferably not more than 5 wt.-%, yet more preferably not more than 4 wt.-%, even more preferably not more than 3 wt.-%, most preferably not more than 2 wt.-%, and in particular not more than 1 wt.-%, in each case relative to the total weight of the reaction mixture, or is essentially free of solvent; preferably the total process is performed essentially in the absence of solvent; preferably the total process is performed essentially in the absence of solvent.

Preferably, the conjugate acid of the organic base has an aqueous pK_{A} value at 25°C of at least 7.0, preferably at least 7.5, more preferably at least 8.0, still more preferably at least 8.5, yet more preferably at least 9.0, even more preferably at least 9.5, most preferably at least 10.0, and in particular at least 10.5.

Preferably, the conjugate acid of the organic base has an aqueous pK_{A} value at 25°C of at most 14.0, preferably at most 13.5, more preferably at most 13.0, still more preferably at most 12.5, yet more preferably at most 12.0, even more preferably at most 11.5, most preferably at most 11.0, and in particular at most 10.5.

Preferably, the organic base is an amine or hydroxylamine; preferably a primary amine, a primary hydroxyl amine, a secondary amine, a secondary hydroxyl amine, a tertiary amine, or a tertiary hydroxyl amine; preferably a di(C₁₋₁₂-alkyl) or a tri(C₁₋₁₂-alkyl)amine; more preferably di-(2-ethylhexyl)amine, triethylamine, tributylamine, or trioctylamine.

Preferably, the organic base is a primary diamine, a secondary diamine, or a tertiary diamine; preferably a tertiary diamine; more preferably (C₁₋₄-alkyl)₂N-(CH₂)₂₋₆-N(C₁₋₄-alkyl)₂; still more preferably tetramethyl ethylene diamine (TMEDA).

Preferably, the organic base is a heteroaromatic compound; preferably an imidazole; more preferably an alkyl imidazole; still more preferably 1-(C₁₋₆-alkyl)-imidazole; yet more preferably 1-methyl imidazole or 1-butyl imidazole.

Preferably, the organic base is a bicyclic compound; preferably a diazabicyclic compound; more preferably 1,8-diazabicyclo[5.4.0]undec-7-en (DBU), 1,5-diazabicyclo[4.3.0]non-5-en (DBN), (1,4-diazabicyclo [2.2.2]octane (DABCO), or the like.

Preferably, the catalyst is selected from (i) trialkyl phosphonium salts; preferably tributyl phosphonium salts; more preferably tributyl phosphonium hydrochlorides; (ii) tetraalkyl phosphonium salts; preferably tetrabutyl phosphonium salts; more preferably tetrabutyl phosphonium chloride; (iii) ammonium salts; and (iv) tetraalkyl ammonium salts; preferably tetrabutyl ammonium salts; more preferably tetrabutyl ammonium chloride.

Preferably, the catalyst forms *in situ* between the base and hydrohalogenic acid that is eliminated in step (b) (autocatalysis).

Preferably, the reaction mixture provided in step (a) is monophasic.

Preferably, the reaction mixture provided in step (a) is biphasic having a first phase and a second phase.

Preferably, the majority of the halogenated 1-butene is contained in the first phase, and the majority of the organic base is contained in the second phase.

Preferably, the reaction mixture obtained in step (b) is monophasic.

Preferably, the reaction mixture obtained in step (b) is biphasic having a first phase and a second phase.

Preferably, the reaction mixture obtained in step (b) is triphasic having a first phase, a second phase, and a third phase.

Preferably, the majority of the halogenated 1,3-butadiene and optionally the majority of residual halogenated 1-butene is contained in the first phase, and the majority of the salt and optionally the majority of residual organic base is contained in the second phase.

Preferably, step (b) is operated as batch-reaction, semi-batch-reaction and continuous reaction.

Preferably, step (b) is performed under boiling conditions.

Preferably, step (b) is performed at a temperature within the range of from -50 to 300°C.

Preferably, step (b) is performed at a temperature within the range of from 20 to 100°C, preferably 20 to 90°C, more preferably 20 to 80°C, still more preferably 20 to 70°C; preferably the halogenated 1-buten is 3,4-dichloro-1-butene (3,4-DBN) and the halogenated 1,3-butadiene is 2-chloro-1,3-butadiene (CP).

Preferably, step (b) is performed at a temperature within the range of from -30 to 10°C, preferably -20 to 10°C, more preferably -10 to 10°C, still more preferably 0 to 10°C; preferably the halogenated 1-buten is 2,3,4-trichloro-1-butene (TCB) and the halogenated 1,3-butadiene is 2,3-dichloro-1,3-butadiene (DCB).

Preferably, step (b) is performed at a pressure within the range of from 0 to 16 bara; preferably 1 to 6 bara.

Preferably, step (b) is performed in a dehydrohalogenation reactor and in a residence time reactor which is arranged downstream of the dehydrohalogenation reactor.

Preferably, the reaction mixture obtained in step (b) is monophasic and in step (c) at least a portion of the halogenated 1,3-butadiene is separated from the reaction mixture by *in-situ* distillation.

Preferably, the reaction mixture obtained in step (b) is biphasic and in step (c) at least a portion of the halogenated 1,3-butadiene is separated from the reaction mixture by *in-situ* distillation.

Preferably, the reaction mixture obtained in step (b) is monophasic and in step (d) the product composition is separated from the salt composition by gas/liquid phase separation, preferably *in-situ* distillation; preferably the halogenated 1-buten is 3,4-dichloro-1-butene (3,4-DBN) and the halogenated 1,3-butadiene is 2-chloro-1,3-butadiene (CP).

Preferably, the reaction mixture obtained in step (b) is biphasic and in step (d) the product composition is separated from the salt composition by liquid/liquid phase separation or solid/liquid phase separation.

Preferably, phase separation involves coalescing separation, centrifuging, decanting, filtration, or any combination thereof.

Preferably, step (d) is operated as batch-reaction, semi-batch-reaction and continuous reaction.

Preferably, in step (d) the product composition is recycled to the reaction mixture.

Preferably, step (d) is performed at a temperature within the range of from -50 to 300°C.

Preferably, step (d) is performed at a temperature within the range of from -40 to 20°C, preferably -30 to 20°C, more preferably -20 to 20°C, still more preferably -10 to 20°C; preferably the halogenated 1-buten is 2,3,4-trichloro-1-butene (TCB) and the halogenated 1,3-butadiene is 2,3-dichloro-1,3-butadiene (DCB).

Preferably, step (d) is performed at a pressure within the range of from 0 to 16 bara; preferably 1 to 6 bara.

Preferably, in step (e) residual halogenated 1-butene is recycled to the reaction mixture.

Preferably, step (f) is operated as batch-reaction, semi-batch-reaction and continuous reaction.

Preferably, step (f) is performed at a temperature within the range of from -50 to 300°C, preferably 0 to 300°C, more preferably 70 to 300°C. For 2-chloro-1,3-butadiene (CP), the temperature range of from 70 to 300°C is particularly preferred, whereas for 2,3-dichloro-1,3-butadiene (DCB) step (f) can also be performed at lower temperature.

Preferably, the base is an amine or hydroxyl amine and wherein step (f) is performed at a temperature that is above the melting temperature of the base under the given conditions.

Preferably, the base is an imidazole and wherein step (f) is performed at a temperature that is above the melting temperature of the base under the given conditions.

Preferably, step (f) is performed at a pressure within the range of from 0 to 16 bara; preferably 0.1 to 6 bara.

Preferably, in step (g) the organic base contained in the organic base composition is purified by rectification.

For the purpose of the specification, a *"low melting halide salt"* is an organic salt having a melting temperature of at most 120°C, preferably at most 115°C, still more preferably at most 110°C, yet more preferably at most 105°C, even more preferably at most 100°C, and most preferably below 100°C.

For the purpose of the specification, an *"ionic liquid"* is defined as a compound completely composed of ions with melting point below 100 °C.

For the purpose of the specification, *"essentially containing no"* means that practically nothing of a certain component is present, whereas it is contemplated that residual amounts of said certain component which might de detectable with sophisticated analytical methods but which have no influence on the overall process may be present.

According to the present invention, it is proposed to alter the process so that instead of the phase transfer agent and inorganic base, an ionic liquid (IL) forming compound, e.g. alkyl imidazole, amine, hydroxy-amines, DABCO, DBU, DBN, TMEDA and the like is used that can capture the cleaved halogen from, e.g., the 3,4-dichloro-1-butene (3,4-DBN) and 2,3,4-trichloro-1-butene (TCB) and form an ionic liquid in the process driving the equilibrium of the initial reaction and simplifying the generation of the (co-)monomers. Exemplary amines are primary amines, primary hydroxyl amines, secondary amines, secondary hydroxyl amines, tertiary amines, or tertiary hydroxyl amines; preferably a di(C₁₋₁₂-alkyl) or a tri(C₁₋₁₂-alkyl)amine; more preferably di-(2-ethylhexyl)amine, triethylamine, tributylamine, or trioctylamine. Preferably, the amine is a primary diamine, a secondary diamine, or a tertiary diamine; preferably a tertiary diamine; more preferably (C₁₋₄-alkyl)₂N-(CH₂)₂₋₆-N(C₁₋₄-alkyl)₂; still more preferably tetramethyl ethylene diamine (TMEDA).

Within all proposed processes, a regeneration of the formed ionic liquid is necessary to recycle the base and to recover the cleaved halogen. The obtained base will be purified within a separate process step.

### Dehydrochlorination (Reactor):

The reaction has an increased reactivity compared to the current dehydrochlorination and can be operated as batch-reaction, semi-batch-reaction and continuous reaction. Both dehydrochlorination (CP, DCB) processes can be conducted between -50 °C and 300 °C, and between 0 bara and 16 bara.

Based on experimental results, current installation, safety related aspects and due to economic reasons, it is preferred to operate the dehydrochlorination process of 2-chloro-1,3-butadiene (CP) production between 20°C and 100°C. A lower temperature means economical disadvantages (reaction has to be cooled by brine or other cooling agents), whereas higher temperatures had shown fast auto-polymerization of 2-chloro-1,3-butadiene (CP) which can be excluded up to 80°C. Moreover, decomposition of 2-chloro-1,3-butadiene (CP) starts around 70°C thereby leading to impurities.

The preferred temperature of the dehydrochlorination of 2,3-dichloro-1,3-butadiene (DCB) is between 0°C and 10°C. This is related to the fact that the stabilizer for 2,3-dichloro-1,3-butadiene (DCB) has high reactivity and needs to be oxidized during the reaction, whereas the flashpoint of 2,3-dichloro-1,3-butadiene (DCB) is 13°C.

The reaction will preferably not be operated below 1 bara, to prevent oxygen leakages into the reactor. Since there is no need of high-pressure during reaction (liquid-liquid system), the max. operating pressure is preferably limited to 6 bara, to avoid high equipment costs.

The experiments had shown that the reaction can be facilitated by comprising a catalyst or adding hydrochloride. The catalyst needs to be inert within later regeneration of ionic liquid to form the base.

The reaction of dehydrochlorination can be accelerated by operation at boiling conditions to separate, e.g., the produced 2-chloro-1,3-butadiene (CP) or 2,3-dichloro-1,3-butadiene (DCB) during reaction process (preferred). An operation without *in-situ* distillation is possible as well.

### Separation:

The selection of the organic base is, among others, dependent on reaction kinetic of dehydrohalogenation. However, for the industrial process a liquid/liquid or gas/liquid phase separation is preferred, so the organic base and ionic liquid, respectively, are preferably selected considering also the physical and chemical parameter for phase separation (e.g. miscibility gap, aggregate state).

The biphasic system containing or essentially consisting of the desired halogenated 1,3-butadiene and ionic liquid is preferably separated mechanically or thermally within a separation process (e.g. coalescing separation, centrifuging, decanting process, filtration, distillation etc.). The separation process can be operated discontinuously and continuously. In general, the process can be operated between the above-mentioned process parameters for dehydrochlorination (T: - 50°C to 300°C; p: 0 bara to 16 bara).

The operating conditions for mechanical separation are dependent on miscibility gap, ionic liquid (aggregate state), economic factors and for brown-field implementation, on infrastructure. For 2,3-dichloro-1,3-butadiene (DCB)/ionic liquid separation a mechanical process is preferred, due to the high ability for auto-polymerization of the monomer and thermal degradation. This process will preferably be operated between -10°C and 20°C with pressures from preferably 0 bara to 6 bara.

A thermal separation process is preferred for 2-chloro-1,3-butadiene (CP)/ionic liquid. The boiling point for 2-chloro-1,3-butadiene (CP) at ambient temperature is approx. 64 °C. Both processes, dehydrochlorination and separation of 2-chloro-1,3-butadiene (CP), can be conducted within one process step. This allows an economic heat management by using the reaction heat for 2-chloro-1,3-butadiene (CP) vaporization but limits the operating conditions for dehydrochlorination.

Thanks to targeted process conditions of separation and dehydrochlorination, fewer side products will be formed within the monomer phase. This leads to a high purity of monomers and therewith a high process efficiency of downstream polymerization.

### Regeneration:

The formed ionic liquid will be discontinuously or continuously either thermally or chemically regenerated by using specific process conditions of preferably -50°C to 300°C, and preferably 0 bara to 16 bara. During the process, a halogenic by-product will be obtained or chemically bound on another substance by decomposing the ionic liquid, forming the originally used base. To reduce the thermal stress of components and to facilitate the recovery process, an equipment with a high specific surface should be used. The base will be recycled by recirculation to dehydrochlorination process. The loss of ionic liquid/base due to thermal and chemical degradation as well as physical losses will be compensated.

The regeneration efficiency/conditions are dependent on the strength of the base and this has an effect on the reaction kinetics of the dehydrochlorination. The desired operational temperature is preferably set to 20°C to 160°C for economic reasons. The operational pressure is preferably between 0 bara to 16 bara.

Depending upon the nature of the base/ionic liquid and the reaction conditions, either a monophasic or a biphasic system is obtained.

Preferred embodiments of the invention are schematically illustrated in Figures 1 to 3. Figure 1 shows a process flow diagram with *in-situ* distillation of produced monomer. A single phase is obtained after dehydrohalogenation. Figure 2 shows a process flow diagram with *in situ* distillation of produced monomer. Two-phases are obtained after dehydrohalogenation. Figure 3 shows a process flow diagram with separation of produced monomer by phase separation.

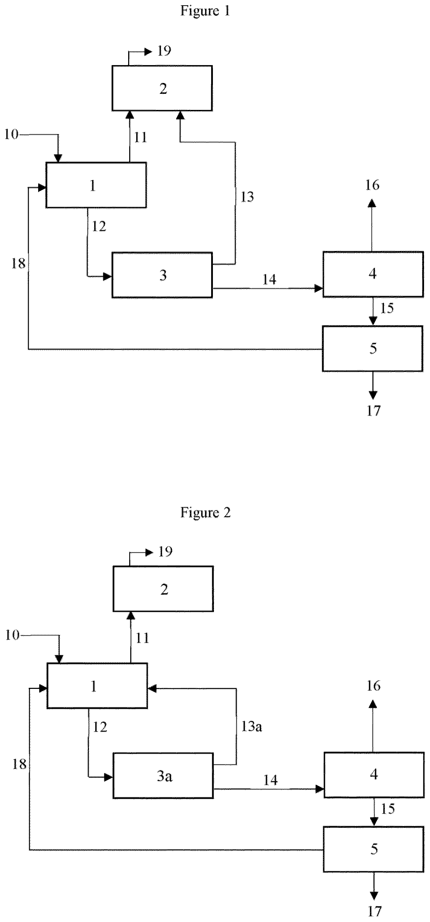

Figure 1 schematically illustrates a preferred embodiment of a process obtaining a monophasic system. Halogenated 1-butene, organic base and optionally catalyst (10) are supplied into a dehydrohalogenation reactor (1) (step (a)). The reaction in step (b) is preferably performed in two stages, namely in dehydrohalogenation reactor (1) and subsequently in a residence time reactor (3) arranged downstream of the dehydrohalogenation reactor (1). Halogenated 1,3-butadiene (11) obtained in the dehydrohalogenation reactor (1) is preferably distilled off *in situ* from the monophasic reaction mixture (step (c)). The remaining residual halogenated 1-butene, halogenated 1,3-butadiene, ionic liquid and residual organic base (12) are then preferably supplied into the residence time reactor (3) for further reaction in step (b). Halogenated 1,3-butadiene (13) obtained in the residence time reactor (3) is preferably also distilled off *in situ* from the monophasic reaction mixture in order to shift the equilibrium and to achieve high conversion (product composition of step (d)). The distilled halogenated 1,3-butadienes (11) and (13) are preferably combined with one another and purified in rectification unit (2) (step (e)) to provide purified halogenated 1,3-butadiene (19). The salt composition containing ionic liquid and residual organic base (14) is preferably supplied to regeneration unit (4) where it is heated to release and separate hydrohalogenic acid (16) from the salt (hydrohalogenic acid composition of step (f)). The remaining organic base (15) (organic base composition of step (f)) is preferably supplied to base purification unit (5) where it is preferably separated from high boiling components (17), preferably by rectification. In a preferred embodiment, the purification unit (5) is operated discontinuously. The thus purified organic base (18) is preferably recycled to the dehydrohalogenation reactor (1) (step (a)).

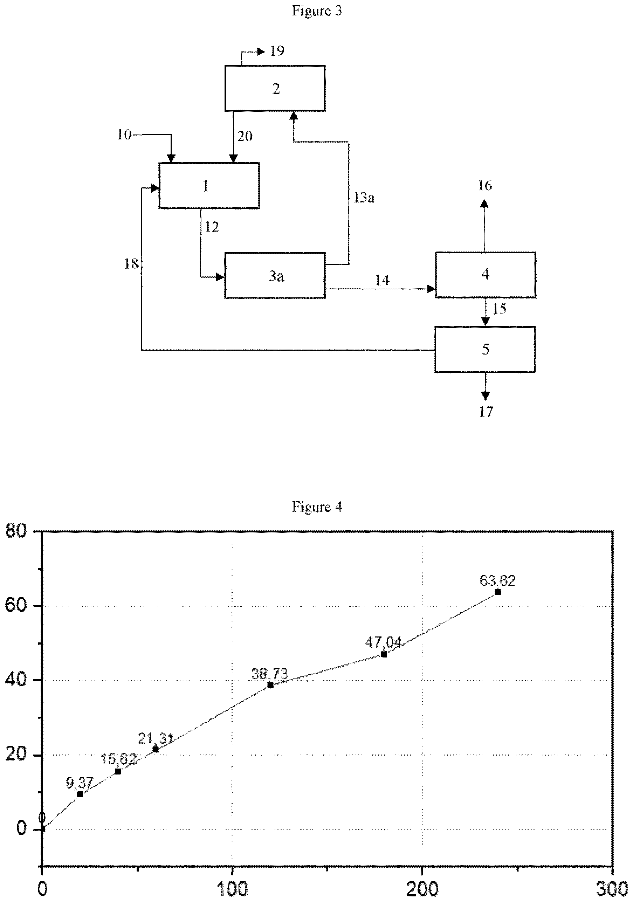

Figures 2 and 3 schematically illustrate preferred embodiments of a process obtaining a biphasic system. The process of Figure 2 involves separation of halogenated 1,3-butadiene by in-situ distillation, the process of Figure 3 involves separation of halogenated 1,3-butadiene by phase separation. Laboratory tests for dehydrohalogenation have shown that an *in situ* distillation is preferred to increase the conversion (Figure 2). For a biphasic system, the reaction is preferably performed in a single stage.

According to the embodiment of Figure 2, halogenated 1,3-butadiene (11) obtained in the dehydrohalogenation reactor (1) is preferably distilled off *in situ* from the monophasic reaction mixture step (c)). In another preferred embodiment, the reaction mixture in step c) is biphasic. The remining residual halogenated 1-butene, halogenated 1,3-butadiene, ionic liquid and residual organic base (12) are then preferably supplied into phase separation unit (3a) where they are separated into a product composition containing halogenated 1,3-butadiene and residual halogenated 1-butene (13a) and a salt composition containing ionic liquid and residual organic base (14). The product composition (13a) is preferably recycled to the dehydrohalogenation reactor (1). The remaining preferred steps correspond to those illustrated for the embodiment of Figure 1.

According to the embodiment of Figure 3, the total amount of the halogenated 1,3-butadiene is contained in the product composition containing halogenated 1,3-butadiene and residual halogenated 1-butene (13a), because no in-situ distillation is performed. After phase separation, the product composition (13a) is purified in rectification unit (2) (step (e)) to provide purified halogenated 1,3-butadiene (19). Residual halogenated 1-butene (20) is preferably recycled to the dehydrohalogenation reactor (1). The remaining preferred steps correspond to those illustrated for the embodiments of Figures 1 and 2.

Further embodiments of the invention are summarized as clauses 1 to 44 here below:
Clause 1: A process for the preparation of a halogenated 1,3-butadiene of general formula (I) 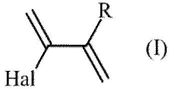 wherein R means -H or Hal; and Hal independently means -Cl, -Br or -I; preferably -Cl; said process comprising the steps of: (a) providing a reaction mixture containing or essentially consisting of - a halogenated 1-butene of general formula (II) 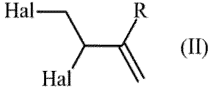 wherein R means -H or Hal; and Hal independently means -Cl, -Br or -I; preferably -Cl; - an organic base; and - optionally a catalyst; (b) eliminating hydrohalogenic acid from the halogenated 1-butene to produce the halogenated 1,3-butadiene; and forming a salt of the eliminated hydrohalogenic acid with the organic base thereby obtaining a low melting halide salt, preferably an ionic liquid, in the reaction mixture; (c) optionally, separating at least a portion of the halogenated 1,3-butadiene from the reaction mixture by in-situ distillation; (d) separating the reaction mixture into - a product composition containing halogenated 1,3-butadiene and optionally residual halogenated 1-butene; and - a salt composition containing salt of the eliminated hydrohalogenic acid with organic base and optionally residual organic base; (e) optionally, purifying the halogenated 1,3-butadiene contained in the product composition; optionally together with the portion of the halogenated 1,3-butadiene optionally separated in step (c); (f) cleaving the salt to release hydrohalogenic acid from the salt and separating the salt composition into - a hydrohalogenic acid composition containing released hydrohalogenic acid; and - an organic base composition containing organic base; (g) optionally, purifying the organic base contained in the organic base composition thereby obtaining purified organic base; and (h) recycling the organic base composition or the purified organic base to the reaction mixture.
Clause 2: The process according to clause 1, wherein the halogenated 1-buten is 3,4-dichloro-1-butene (3,4-DBN) and the halogenated 1,3-butadiene is 2-chloro-1,3-butadiene (CP): 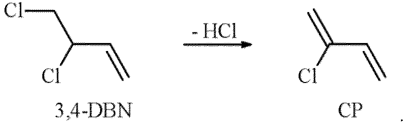
Clause 3: The process according to clause 1, wherein the halogenated 1-buten is 2,3,4-trichloro-1-butene (TCB) and the halogenated 1,3-butadiene is 2,3-dichloro-1,3-butadiene (DCB): 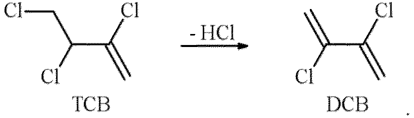
Clause 4: The process according to any of the preceding clauses, wherein the reaction mixture has a water content of not more than 8 wt.-%, preferably not more than 7 wt.-%, more preferably not more than 6 wt.-%, still more preferably not more than 5 wt.-%, yet more preferably not more than 4 wt.-%, even more preferably not more than 3 wt.-%, most preferably not more than 2 wt.-%, and in particular not more than 1 wt.-%, in each case relative to the total weight of the reaction mixture, or is essentially free of water; preferably wherein the total process is performed essentially in the absence of water. Clause 5: The process according to any of the preceding clauses, wherein the reaction mixture has a solvent content of not more than 8 wt.-%, preferably not more than 7 wt.-%, more preferably not more than 6 wt.-%, still more preferably not more than 5 wt.-%, yet more preferably not more than 4 wt.-%, even more preferably not more than 3 wt.-%, most preferably not more than 2 wt.-%, and in particular not more than 1 wt.-%, in each case relative to the total weight of the reaction mixture, or is essentially free of solvent; preferably wherein the total process is performed essentially in the absence of solvent; preferably wherein the total process is performed essentially in the absence of solvent.
Clause 6: The process according to any of the preceding clauses, wherein the conjugate acid of the organic base has an aqueous pK_{A} value at 25°C of at least 7.0, preferably at least 7.5, more preferably at least 8.0, still more preferably at least 8.5, yet more preferably at least 9.0, even more preferably at least 9.5, most preferably at least 10.0, and in particular at least 10.5.
Clause 7: The process according to any of the preceding clauses, wherein the conjugate acid of the organic base has an aqueous pK_{A} value at 25°C of at most 14.0, preferably at most 13.5, more preferably at most 13.0, still more preferably at most 12.5, yet more preferably at most 12.0, even more preferably at most 11.5, most preferably at most 11.0, and in particular at most 10.5.
Clause 8: The process according to any of the preceding clauses, wherein the organic base is an amine or hydroxylamine; preferably a primary amine, a primary hydroxyl amine, a secondary amine, a secondary hydroxyl amine, a tertiary amine, or a tertiary hydroxyl amine; preferably a di(C₁₋₁₂-alkyl) or a tri(C₁₋₁₂-alkyl)amine; more preferably di-(2-ethylhexyl)amine, triethylamine, tributylamine, or trioctylamine.
Clause 9: The process according to any of the preceding clauses, wherein the organic base is a primary diamine, a secondary diamine, or a tertiary diamine; preferably a tertiary diamine; more preferably (C₁₋₄-alkyl)₂N-(CH₂)₂₋₆-N(C₁₋₄-alkyl)₂; still more preferably tetramethyl ethylene diamine (TMEDA).
Clause 10: The process according to any of the preceding clauses, wherein the organic base is a heteroaromatic compound; preferably an imidazole; more preferably an alkyl imidazole; still more preferably 1-(C₁₋₆-alkyl)-imidazole; yet more preferably 1-methyl imidazole or 1-butyl imidazole.
Clause 11: The process according to any of the preceding clauses, wherein the organic base is a bicyclic compound; preferably a diazabicyclic compound; more preferably 1,8-diazabicyclo[5.4.0]undec-7-en (DBU), 1,5-diazabicyclo[4.3.0]non-5-en (DBN), (1,4-diazabicyclo [2.2.2]octane (DABCO), or the like.
Clause 12: The process according to any of the preceding clauses, wherein the catalyst is selected from (i) trialkyl phosphonium salts; preferably tributyl phosphonium salts; more preferably tributyl phosphonium hydrochlorides; (ii) tetraalkyl phosphonium salts; preferably tetrabutyl phosphonium salts; more preferably tetrabutyl phosphonium chloride; (iii) ammonium salts; and (iv) tetraalkyl ammonium salts; preferably tetrabutyl ammonium salts; more preferably tetrabutyl ammonium chloride.
Clause 13: The process according to any of the preceding clauses, wherein the catalyst forms *in situ* between the base and hydrohalogenic acid that is eliminated in step (b) (autocatalysis).
Clause 14: The process according to any of the preceding clauses, wherein the reaction mixture provided in step (a) is monophasic.
Clause 15: The process according to any of clauses 1 to 13, wherein the reaction mixture provided in step (a) is biphasic having a first phase and a second phase.
Clause 16: The process according to clause 15, wherein - the majority of the halogenated 1-butene is contained in the first phase, and - the majority of the organic base is contained in the second phase.
Clause 17: The process according to any of the preceding clauses, wherein the reaction mixture obtained in step (b) is monophasic.
Clause 18: The process according to any of clauses 1 to 16, wherein the reaction mixture obtained in step (b) is biphasic having a first phase and a second phase.
Clause 19: The process according to any of clauses 1 to 16, wherein the reaction mixture obtained in step (b) is triphasic having a first phase, a second phase, and a third phase.
Clause 20: The process according to clause 18 or 19, wherein - the majority of the halogenated 1,3-butadiene and optionally the majority of residual halogenated 1-butene is contained in the first phase, and - the majority of the salt and optionally the majority of residual organic base is contained in the second phase.
Clause 21: The process according to any of the preceding clauses, wherein step (b) is operated as batch-reaction, semi-batch-reaction and continuous reaction.
Clause 22: The process according to any of the preceding clauses, wherein step (b) is performed under boiling conditions.
Clause 23: The process according to any of the preceding clauses, wherein step (b) is performed at a temperature within the range of from -50 to 300°C.
Clause 24: The process according to clause 23, wherein step (b) is performed at a temperature within the range of from 20 to 100°C, preferably 20 to 90°C, more preferably 20 to 80°C, still more preferably 20 to 70°C; preferably wherein the halogenated 1-buten is 3,4-dichloro-1-butene (3,4-DBN) and the halogenated 1,3-butadiene is 2-chloro-1,3-butadiene (CP).
Clause 25: The process according to clause 23, wherein step (b) is performed at a temperature within the range of from -30 to 10°C, preferably -20 to 10°C, more preferably -10 to 10°C, still more preferably 0 to 10°C; preferably wherein the halogenated 1-buten is 2,3,4-trichloro-1-butene (TCB) and the halogenated 1,3-butadiene is 2,3-dichloro-1,3-butadiene (DCB).
Clause 26: The process according to any of the preceding clauses, wherein step (b) is performed at a pressure within the range of from 0 to 16 bara; preferably 1 to 6 bara.
Clause 27: The process according to any of the preceding clauses, wherein step (b) is performed in a dehydrohalogenation reactor and in a residence time reactor which is arranged downstream of the dehydrohalogenation reactor.
Clause 28: The process according to any of the preceding clauses, wherein the reaction mixture obtained in step (b) is monophasic and in step (c) at least a portion of the halogenated 1,3-butadiene is separated from the reaction mixture by in-situ distillation.
Clause 29: The process according to any of clauses 1 to 27, wherein the reaction mixture obtained in step (b) is biphasic and in step (c) at least a portion of the halogenated 1,3-butadiene is separated from the reaction mixture by in-situ distillation.
Clause 30: The process according to any of the preceding clauses, wherein the reaction mixture obtained in step (b) is monophasic and in step (d) the product composition is separated from the salt composition by gas/liquid phase separation, preferably in-situ distillation; preferably wherein the halogenated 1-buten is 3,4-dichloro-1-butene (3,4-DBN) and the halogenated 1,3-butadiene is 2-chloro-1,3-butadiene (CP).
Clause 31: The process according to any of the preceding clauses, wherein the reaction mixture obtained in step (b) is biphasic and in step (d) the product composition is separated from the salt composition by liquid/liquid phase separation or solid/liquid phase separation.
Clause 32: The process according to clause 31, wherein phase separation involves coalescing separation, centrifuging, decanting, filtration, or any combination thereof.
Clause 33: The process according to any of the preceding clauses, wherein step (d) is operated as batch-reaction, semi-batch-reaction and continuous reaction.
Clause 34: The process according to any of the preceding clauses, wherein in step (d) the product composition is recycled to the reaction mixture.
Clause 35: The process according to any of the preceding clauses, wherein step (d) is performed at a temperature within the range of from -50 to 300°C.
Clause 36: The process according to clause 35, wherein step (d) is performed at a temperature within the range of from -40 to 20°C, preferably -30 to 20°C, more preferably -20 to 20°C, still more preferably -10 to 20°C; preferably wherein the halogenated 1-buten is 2,3,4-trichloro-1-butene (TCB) and the halogenated 1,3-butadiene is 2,3-dichloro-1,3-butadiene (DCB).
Clause 37: The process according to any of the preceding clauses, wherein step (d) is performed at a pressure within the range of from 0 to 16 bara; preferably 1 to 6 bara.
Clause 38: The process according to any of the preceding clauses, wherein in step (e) residual halogenated 1-butene is recycled to the reaction mixture.
Clause 39: The process according to any of the preceding clauses, wherein step (f) is operated as batch-reaction, semi-batch-reaction and continuous reaction.
Clause 40: The process according to any of the preceding clauses, wherein step (f) is performed at a temperature within the range of from -50 to 300°C, preferably 0 to 300°C, more preferably 70 to 300°C.
Clause 41: The process according to clause 40, wherein the base is an amine or hydroxyl amine and wherein step (f) is performed at a temperature that is above the melting temperature of the base under the given conditions.
Clause 42: The process according to clause 40, wherein the base is an imidazole and wherein step (f) is performed at a temperature that is above the melting temperature of the base under the given conditions.
Clause 43: The process according to any of the preceding clauses, wherein step (f) is performed at a pressure within the range of from 0 to 16 bara; preferably 0.1 to 6 bara.
Clause 44: The process according to any of the preceding clauses, wherein in step (g) the organic base contained in the organic base composition is purified by rectification.

The following examples further illustrate the invention but are not to be construed as limiting its scope:

### Example 1 dehydrochlorination of 3,4-dichloro-1-butene to afford 2-chloro-1,3-butadiene:

The following experiments have been conducted with 3,4-dichloro-1-butene (3,4-DBN):

| Exp. | 3,4-DBN | | base | | | catalyst | | temp | time | | phase separation | | conversion |
|---|---|---|---|---|---|---|---|---|---|---|---|---|---|
| | mol | g | nature | mol | g | nature | g | °C | h | min | start | end | % |
| 1-1 | 0.2 | 25 | NOct3 | 0.2 | 70.74 | none | 0 | 60 | 4 | | no | no | 0 |
| 1-2 | 0.2 | 25 | TMEDA | 0.1 | 11.62 | none | 0 | 23 | 2 | | no | solid | 0.9 |
| 1-3 | 0.2 | 25 | TMEDA | 0.1 | 11.62 | Bu4PCl | 4 | 23 | 2 | | no | yes | 0.2 |
| 1-4 | 0.2 | 25 | TMEDA | 0.1 | 11.62 | Bu4PCl | 4 | 40 | | 30 | no | yes | 15.9 |
| 1-5 | 0.2 | 25 | TMEDA | 0.1 | 11.62 | none | 0 | 40 | | 30 | no | yes | 0.5 |
| 1-6 | 0.2 | 25 | TMEDA | 0.1 | 11.62 | Bu4PCl | 4 | 60 | | 30 | no | solid | 3.1 |
| 1-7 | 0.2 | 25 | NEt3 | 0.2 | 20.24 | none | 0 | 60 | 24 | | no | yes | 3.8 |
| 1-8 | 0.2 | 25 | NEt3 | 0.2 | 20.24 | HCl | 0 | 60 | 24 | | no | no | 13.4 |
| 1-9 | 0.2 | 25 | NEt3 | 0.4 | 40.48 | none | 0 | 60 | 24 | | no | no | 24.1 |
| 1-10 | 0.2 | 25 | NEt3 | 0.2 | 20.24 | Bu4PCl | 4 | 23 | 24 | | no | yes | 5.3 |
| 1-11 | 0.2 | 25 | NEt3 | 0.2 | 20.24 | Bu4PCl | 4 | 100 | | 15 | no | yes | <27.2 |
| 1-12 | 0.2 | 25 | NEt3 | 0.2 | 20.24 | none | 0 | 100 | 2 | | no | ves | 9.5 |
| 1-13 | 0.2 | 25 | NEt3 | 0.2 | 20.24 | Bu4PCl | 4 | 100 | | 20 | no | yes | 18.4 |
| 1-14 | 0.2 | 25 | NBut3 | 0.4 | 74.12 | none | 0 | 60 | 4 | | no | yes | 0.6 |
| 1-15 | 0.2 | 25 | NBut3 | 0.4 | 74.12 | Bu4PCl | 6 | 60 | 4 | | no | no | 4.6 |
| 1-16 | 0.2 | 25 | NBut3 | 0.4 | 74.12 | Bu4PCl | 8 | 80 | 4 | | no | no | 40.8 |
| 1-17 | 0.2 | 25 | NBut3 | 0.4 | 74.12 | Bu4PCl | 8 | 60 | 24 | | no | yes | 3.9 |
| 1-18 | 0.2 | 25 | NBut3 | 0.2 | 37.06 | Bu4PCl | 4 | 150 | 1 | | yes | no | <21.6 |
| 1-19 | 0.2 | 25 | DBU | 0.2 | 30.44 | none | 0 | 23 | 0 | 0 | no | yes | # |
| 1-20 | 0.2 | 25 | DBU | 0.2 | 30.44 | none | 0 | 10 | 1 | | no | yes | 56.4 |
| 1-21 | 0.2 | 25 | MeIm | 0.2 | 16.4 | Bu4PCl | 4 | 60 | 4 | | no | yes | 10.4 |
| 1-22 | 0.2 | 25 | MeIm | 0.2 | 16.4 | Bu4PCl | 4 | 110 | | 25 | no | yes | 12.6 |
| 1-23 | 0.2 | 25 | MeIm | 0.2 | 16.4 | none | 0 | 100 | 2 | | no | yes | 21.0 |
| 1-24 | 0.2 | 25 | MeIm | 0.2 | 16.4 | Bu4PCl | 4 | 100 | 1 | | no | yes | 20.9 |
| 1-25 | 0.2 | 25 | BuIm | 0.4 | 49.64 | Bu4PCl | 6 | 60 | 24 | | no | no | 34.2 |
| 1-26 | 0.2 | 25 | BuIm | 0.4 | 49.64 | Bu4PCl | 8 | 80 | 24 | | no | yes | 69.8 |
| 1-27 | 0.1 | 12.5 | NEt3 | 0.2 | 20.24 | Bu4PCl | 8 | 60 | 24 | | no | yes | 81.1 |
| 1-28 | 0.1 | 12.5 | MeIm | 0.2 | 16.4 | Bu4PCl | 4 | 40 | 4 | | no | no | 1.3 |
| 1-29 | 0,2 | 25 | BuIm | 0,4 | 49,64 | Bu4PCl | 4 | 80 | 4 | | no | no | 31.2 |
| 1-30 | 0,2 | 25 | BuIm | 0,4 | 49,64 | Bu4PCl | 0 | 80 | 4 | | no | no | 23.5 |
| 1-31 | 0,2 | 25 | BuIm | 0,2 | 24,82 | Bu4PCl | 4 | 80 | 4 | | no | no | 18.7 |
| 1-32 | 0,2 | 25 | BuIm | 0,4 | 49,64 | Bu4PCl | 4 | 60 | 4 | | no | no | 6.4 |
| 1-33 | 0.2 | 25 | MeIm | 0.4 | 32,8 | Bu4PCl | 4 | 60 | 4 | | no | yes | 10.4 |
| 1-34 | 0.2 | 25 | MeIm | 0.2 | 16.4 | Bu4PCl | 2 | 60 | 4 | | no | yes | 4.8 |
| 1-35 | 0,2 | 25 | Di-2-EHA | 0,4 | 96,6 | Bu4PCl | 4 | 60 | 4 | | no | no | 14.5 |
| 1-36 | 02 | 25 | Di-2-EHA | 02 | 48,3 | Bu4PCl | 4 | 60 | 4 | | no | no | 8.7 |
| 1-37 | 0,2 | 25 | Di-2-EHA | 0,4 | 96,6 | Bu4PCl | 2 | 60 | 4 | | no | no | 6.7 |
| 1-38 | 0,2 | 25 | Di-2-EHA | 0,2 | 48,3 | Bu4PCl | 4 | 80 | 4 | | no | no | 18.0 |

| | | | | | | | | | | | | | |
|---|---|---|---|---|---|---|---|---|---|---|---|---|---|
| NOct3: trioctylamine; TMEDA: tetramethyl ethylene diamine; NEt3: triethylamine; NBut3: tributylamine; Di-2-EHA: di-(2-ethylhexl)amine; DBU: 1,8-diazabicyclo[5.4.0]undec-7-en; MeIm: 1-methyl imidazole; BuIm: 1-butyl imidazole; Bu4PCl: tetrabutyl phosphonium chloride. #: Very strong reaction without any analysis due to solids | | | | | | | | | | | | | |

Figure 4 shows the reaction kinetics of Example 1-27, which was conducted at 60 °C without distillation.

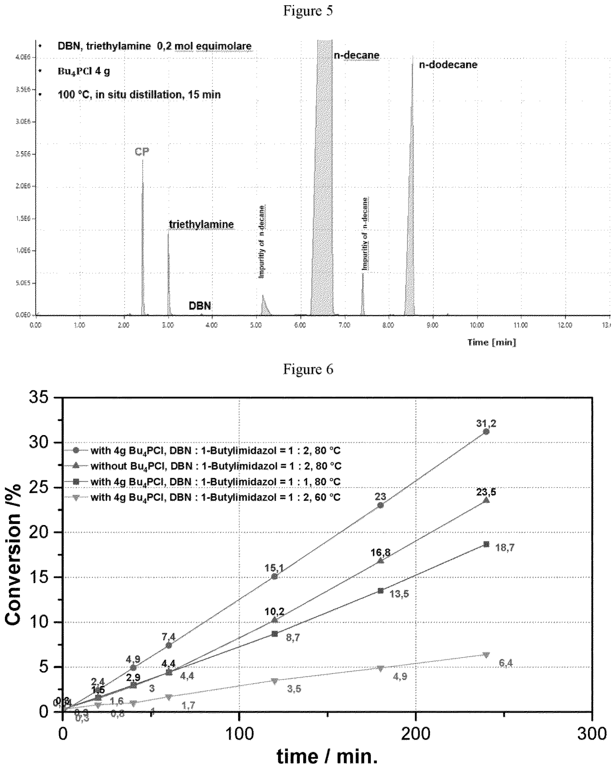

The product of Example 1-11 (Figure 5) was analyzed by gas chromatography showing the peak of 2-chloro-1,3-butadiene (CP), 3,4-dichloro-1-butene (3,4-DBN) and triethylamine (NEt3) as well as the external standard. Two small peaks indicate the impurities that may result from raw material or reaction.

With respect to Examples 1-22, the distillate was analyzed without finding residual organic base methylimidazole (MeIm).

Examples 1-7 and 1-8 (3,4-DBN) demonstrate that in specific cases adding hydrochloric acid has the same effect as a catalyst.

Examples 1-19 and 1-20 show a strong reactivity when employing strong organic bases (DBU). However, when using such strong organic bases, regeneration of ionic liquids will be difficult because hydrohalogenic acid will not be easily released upon heating. With respect to Example 1-19 specifically, no analysis was performed due to solids.

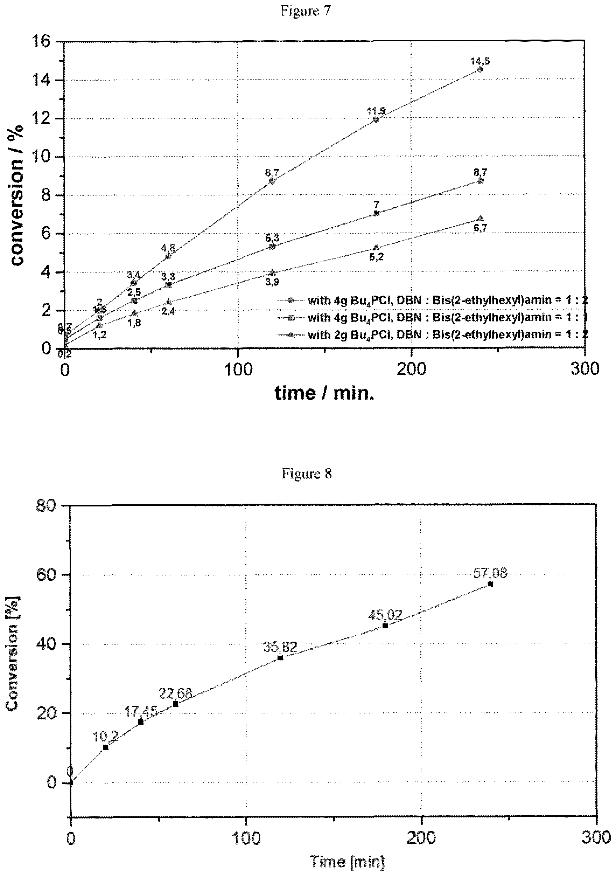

Examples 1-29 & 1-30 (Figure 6) and 1-35 & 1-37 (Figure 7) shows a strong reactivity increase by comprising a catalyst on the reaction. The same effect has been proven by comprising MeIm (1-21 & 1-34). The final conversion (MeIm) doesn't increase while using a catalyst.

Examples 1-29 & 1-31 and 1-35 & 1-36 shows that an increase in base to DBN ratio leads to higher reactivity when comprising Bulm (Figure 6) or Di-2-EHA (Figure 7) whereas this shows no effect when comprising MeIm 1-21 & 1-33.

Examples 1-29 & 1-32 (Figure 6) and 1-36 & 1-38 (Figure 7) shows that the temperature is important for reactivity.

### Example 2:

The following experiments have been conducted with 2,3,4-trichloro-1-butene (TCB):

| Exp. | TCB | | base | | | catalyst | | temp | time | | phase separation | | conversion |
|---|---|---|---|---|---|---|---|---|---|---|---|---|---|
| | mol | g | nature | mol | g | nature | g | °C | h | min | start | end | % |
| 2-1 | 0.1 | 16 | MeIm | 0.1 | 8.2 | Bu4PCl | 3 | 78 | 4 | | yes | yes | 75.1 |
| 2-2 | 0.1 | 16 | MeIm | 0.1 | 8.2 | Bu4PCl | 2 | 78 | 4 | | yes | yes | 74 |
| 2-3 | 0.1 | 16 | MeIm | 0.1 | 8.2 | Bu4PCl | 1 | 78 | 4 | | yes | yes | 64.5 |
| 2-4 | 0.1 | 16 | NBut3 | 0.1 | 18.53 | Bu4PCl | 2 | 78 | 4 | | yes | yes | 24.9 |
| 2-5 | 0.2 | 32 | MeIm | 0.2 | 16.4 | Bu4PCl | 4 | 60 | 4 | | no | yes | 57.9 |
| 2-6 | 0.2 | 32 | MeIm | 0.2 | 16.4 | none | 0 | 60 | 4 | | no | yes | 57.2 |
| 2-7 | 0.2 | 32 | NEt3 | 0.2 | 20.48 | Bu4PCl | 4 | 60 | | 30 | ves | solid | 19 |
| 2-8 | 0.2 | 32 | NEt3 | 0.2 | 37.06 | Bu4PCl | 8 | 10 | 72 | | no | yes | 48.9 |

| | | | | | | | | | | | | | |
|---|---|---|---|---|---|---|---|---|---|---|---|---|---|
| NEt3: triethylamine; NBut3: tributylamine; MeIm: 1-methyl imidazole; Bu4PCl: tetrabutyl phosphonium chloride | | | | | | | | | | | | | |

Figure 8 shows the development of conversion in percent over time in minutes with respect to dehydrochlorination of 2,3,4-trichloro-1-butene (TCB) for Example 2-5.

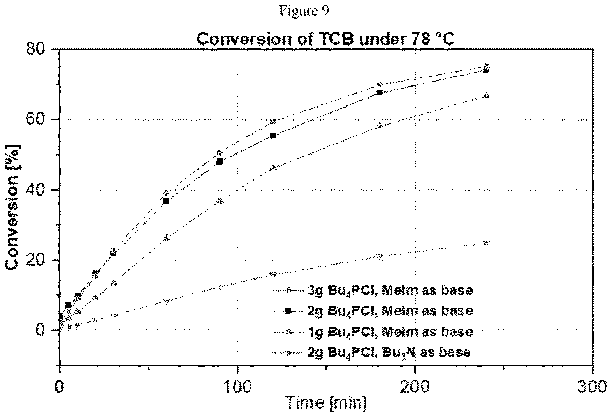

Example 2-1, 2-2 & 2-3 (Figure 9) show a reactivity increase by comprising a catalyst on the reaction.

According to the extensive experiments related to dehydrochlorination providing 2-chloro-1,3-butadiene (CP) and 2,3-dichloro-1,3-butadiene (DCB), different organic bases have different utility in the two processes. There is indication that the 2-chloro-1,3-butadiene (CP) synthesis delivers advantages by comprising amines as organic base. Other organic bases can be used as well.

Summing up, the in-situ distillation seems to have advantages for many bases (e.g. methylimidazole). For bases like tributylamine and triethylamine, a multistage distillation (rectification process) would be necessary.

### Reference numerals:

| | | | |
|---|---|---|---|
| 1 | dehydrohalogenation reactor | 13 | halogenated 1,3-butadiene |
| 2 | rectification unit | 13a | halogenated 1,3-butadiene + residual educt |
| 3 | residence time reactor | 14 | ionic liquid + residual organic base |
| 3a | phase separation unit | 15 | organic base |
| 4 | regeneration unit | 16 | hydrohalogenic acid |
| 5 | base purification unit | 17 | high boiling components |
| 10 | halogenated 1-butene + organic base | 18 | organic base |
| 11 | halogenated 1,3-butadiene | 19 | halogenated 1,3-butadiene |
| 12 | residual halogenated 1-butene + halogenated 1,3-butadiene + ionic liquid + residual organic base | 20 | residual halogenated 1-butene |

## Claims

1. A process for the preparation of a halogenated 1,3-butadiene of general formula (I) 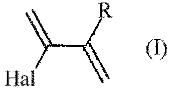 wherein
R means -H or Hal; and
Hal independently means -Cl, -Br or -I; preferably -Cl;
said process comprising the steps of:
(a) providing a reaction mixture containing or essentially consisting of
- a halogenated 1-butene of general formula (II) 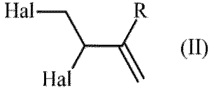 wherein
R means -H or Hal; and
Hal independently means -Cl, -Br or -I; preferably -Cl;
- an organic base; and
- optionally a catalyst;
(b) eliminating hydrohalogenic acid from the halogenated 1-butene to produce the halogenated 1,3-butadiene; and forming a salt of the eliminated hydrohalogenic acid with the organic base thereby obtaining a low melting halide salt, preferably an ionic liquid, in the reaction mixture;
(c) optionally, separating at least a portion of the halogenated 1,3-butadiene from the reaction mixture by in-situ distillation;
(d) separating the reaction mixture into
- a product composition containing halogenated 1,3-butadiene and optionally residual halogenated 1-butene; and
- a salt composition containing salt of the eliminated hydrohalogenic acid with organic base and optionally residual organic base;
(e) optionally, purifying the halogenated 1,3-butadiene contained in the product composition; optionally together with the portion of the halogenated 1,3-butadiene optionally separated in step (c);
(f) cleaving the salt to release hydrohalogenic acid from the salt and separating the salt composition into
- a hydrohalogenic acid composition containing released hydrohalogenic acid; and
- an organic base composition containing organic base;
(g) optionally, purifying the organic base contained in the organic base composition thereby obtaining purified organic base; and
(h) recycling the organic base composition or the purified organic base to the reaction mixture.

2. The process according to claim 1, wherein
(i) the halogenated 1-buten is 3,4-dichloro-1-butene (3,4-DBN) and the halogenated 1,3-butadiene is 2-chloro-1,3-butadiene (CP): 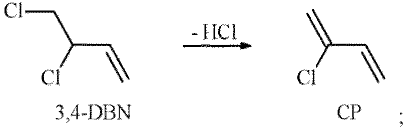 or
(ii) the halogenated 1-buten is 2,3,4-trichloro-1-butene (TCB) and the halogenated 1,3-butadiene is 2,3-dichloro-1,3-butadiene (DCB):

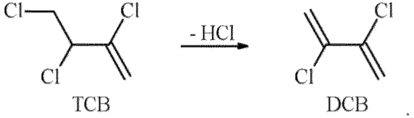

3. The process according to any of the preceding claims, wherein the organic base is a primary diamine, a secondary diamine, or a tertiary diamine; preferably a tertiary diamine; more preferably (C₁₋₄-alkyl)₂N-(CH₂)₂₋₆-N(C₁₋₄-alkyl)₂; still more preferably tetramethyl ethylene diamine (TMEDA).

4. The process according to any of the preceding claims, wherein the organic base is a heteroaromatic compound; preferably an imidazole; more preferably an alkyl imidazole; still more preferably 1-(C₁₋₆-alkyl)-imidazole; yet more preferably 1-methyl imidazole or 1-butyl imidazole.

5. The process according to any of the preceding claims, wherein the organic base is a bicyclic compound; preferably a diazabicyclic compound; more preferably 1,8-diazabicyclo[5.4.0]undec-7-en (DBU), 1,5-diazabicyclo[4.3.0]non-5-en (DBN), (1,4-diazabicyclo [2.2.2]octane (DABCO), or the like.

6. The process according to any of the preceding claims, wherein the catalyst is selected from
(i) trialkyl phosphonium salts; preferably tributyl phosphonium salts; more preferably tributyl phosphonium hydrochlorides;
(ii) tetraalkyl phosphonium salts; preferably tetrabutyl phosphonium salts; more preferably tetrabutyl phosphonium chloride;
(iii) ammonium salts; and
(iv) tetraalkyl ammonium salts; preferably tetrabutyl ammonium salts; more preferably tetrabutyl ammonium chloride.

7. The process according to any of the preceding claims, wherein the reaction mixture provided in step (a) is monophasic.

8. The process according to any of claims 1 to 6, wherein the reaction mixture provided in step (a) is biphasic or triphasic, preferably biphasic.

9. The process according to any of the preceding claims, wherein step (b) is performed at a temperature within the range of from -50 to 300°C.

10. The process according to any of the preceding claims, wherein the reaction mixture obtained in step (b) is monophasic and in step (c) at least a portion of the halogenated 1,3-butadiene is separated from the reaction mixture by in-situ distillation.

11. The process according to any of the preceding claims, wherein the reaction mixture obtained in step (b) is monophasic and in step (d) the product composition is separated from the salt composition by gas/liquid phase separation, preferably in-situ distillation; preferably wherein the halogenated 1-buten is 3,4-dichloro-1-butene (3,4-DBN) and the halogenated 1,3-butadiene is 2-chloro-1,3-butadiene (CP).

12. The process according to any of the preceding claims, wherein step (d) is performed at a temperature within the range of from -50 to 300°C.

13. The process according to any of the preceding claims, wherein in step (e) residual halogenated 1-butene is recycled to the reaction mixture.

14. The process according to any of the preceding claims, wherein step (f) is performed at a temperature within the range of from -50 to 300°C, preferably 0 to 300°C, more preferably 70 to 300°C.

15. The process according to any of the preceding claims, wherein in step (g) the organic base contained in the organic base composition is purified by rectification.
